# EUROPEAN PATENT APPLICATION

(11) **EP 4 014 970 A1**
(43) Date of publication of application: **22.06.2022**
(21) Application number: 20386059.8
(22) Date of filing: 21.12.2020
(51) Int. Cl.: A61K 9/20, A61K 31/4152, A61P 7/04

(54) **A SOLID ORAL COMPOSITION OF ELTROMBOPAG OLAMINE**

(71) Applicant: Genepharm S.A., 15351 Pallini (GR)
(72) Inventor: Xenogiorgis, Vasileios, 17676 Kallithea Athens (GR); Mpenekis, Vasilis, 16341 Ilioupoli Athens (GR)
(74) Representative: Roukounas, Dimitrios

(57) **Abstract**

A solid oral composition comprising 5-40% by weight of 3'-[(2Z)-[1-(3,4-dimethylphenyl)-1,5-dihydro-3-methyl-5-oxo-4H-pyrazol-4-ylidene]hydrazino]-2'-hydroxy-[1,1'-biphenyl]-3-carboxylic acid bis-(monoethanolamine) and at least one excipient; wherein the composition is prepared by direct compression.

## Description

### FIELD OF THE INVENTION

The present invention relates to a solid oral composition of 3'-[(2Z)-[1-(3,4-dimethylphenyl)-1,5-dihydro-3-methyl-5-oxo-4H-pyrazol-4-ylidene]hydrazino]-2'-hydroxy-[1,1'-biphenyl]-3-carboxylic acid bis-(monoethanolamine) {referred to as eltrombopag olamine}, prepared by direct compression.

### BACKGROUND OF THE INVENTION

Eltrombopag olamine is a small molecule thrombopoietin (TPO) receptor agonist useful in treating thrombocytopenia in patients with chronic ITP, thrombocytopenia in patients with hepatitis C infection and severe aplastic anemia.

**European Patent** 2152237B1 (assigned to Smithkline Beecham Corporation, referred to herein as '237) teaches novel tablets of eltrombopag olamine using diluents(s) substantially free of coordinating metals and/or reducing sugars. The inventors of '237 found wet granulation process particularly suitable for providing high strength, low breakage tablets comprising relatively high concentrations of eltrombopag olamine. However, the scalability of wet granulation requires a larger amount of trials in order to optimize the granulation regime (e.g. impeller speed, chopper speed, pump speed, endpoint, step-wise granulation times) and equipments such as high shear granulators, roller compactors, hot melt extruders or other higher energy consuming equipments followed by dryers.

The present invention relates to solid oral compositions of eltrombopag olamine prepared by direct compression which will avoid additional equipments such as granulators and dryers. These tablets have low breakage and high strength for relatively high concentrations of eltrombopag olamine.

**European** application publication EP3041511A2 (assigned to Hetero Research Foundation, referred to herein as '511) teaches preparation of tablets with high disintegrant amounts using wet granulation process. However, the present invention relates solid oral compositions of eltrombopag olamine prepared by direct compression.

PCT publication WO 2019071111A1 (assigned to Teva, referred to herein as '111) claims choline salt of eltrombopag. However, the present invention relates to solid oral compositions of the olamine salt of eltrombopag.

### OBJECT OF THE INVENTION

The object of the present invention is to provide solid oral compositions of 3'-[(2Z)-[1-(3,4-dimethylphenyl)-1,5-dihydro-3-methyl-5-oxo-4H-pyrazol-4-ylidene]hydrazino]-2'-hydroxy-[1,1'-biphenyl]-3-carboxylic acid bis-(monoethanolamine) prepared by simple process of direct compression.

A further object of the present invention is to provide solid oral compositions of 3'-[(2Z)-[1-(3,4-dimethylphenyl)-1,5-dihydro-3-methyl-5-oxo-4H-pyrazol-4-ylidene]hydrazino]-2'-hydroxy-[1,1'-biphenyl]-3-carboxylic acid bis-(monoethanolamine) which are stable when prepared by direct compression.

### SUMMARY OF THE INVENTION

A solid oral composition comprising 5-40% by weight of 3'-[(2Z)-[1-(3,4-dimethylphenyl)-1,5-dihydro-3-methyl-5-oxo-4H-pyrazol-4-ylidene]hydrazino]-2'-hydroxy-[1,1'-biphenyl]-3-carboxylic acid bis-(monoethanolamine) and at least one excipient; wherein the composition is prepared by direct compression.

### BRIEF DESCRIPTION OF THE DRAWINGS

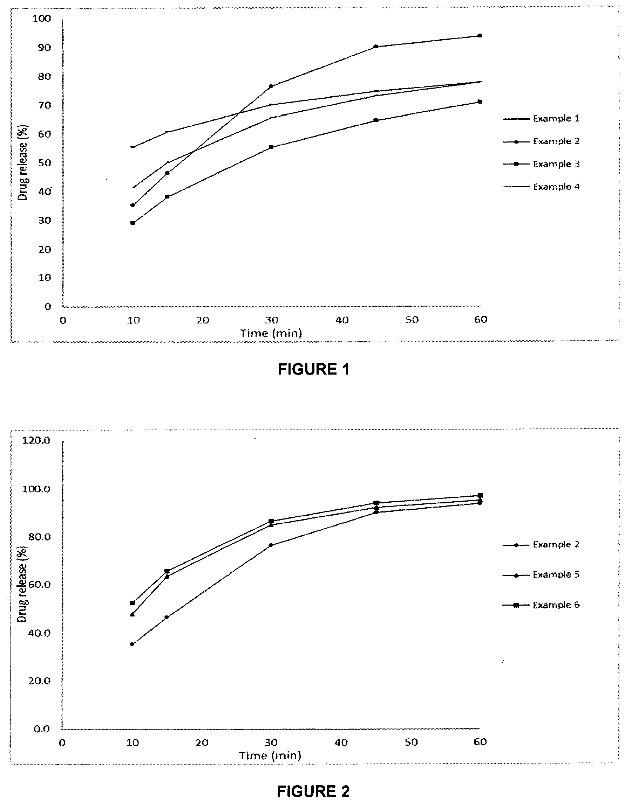

**Figure 1** : Comparative dissolution profiles of Examples 1-2
**Figure 2** : Comparative dissolution profiles of Examples 5-6

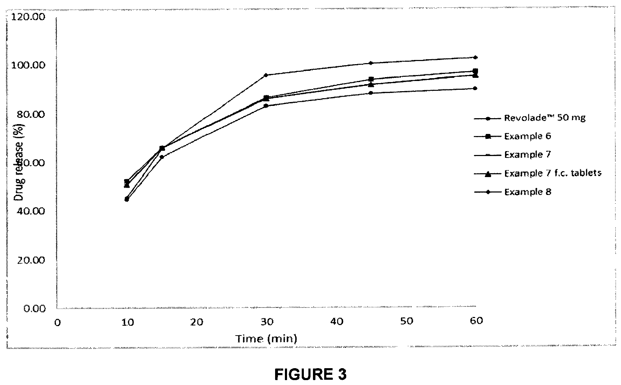

**Figure 3****:** Comparative dissolution profiles of Examples 6-8

### DESCRIPTION OF THE INVENTION

It has surprisingly been found by the present inventors, that the inventive solid oral composition comprising 3'-[(2Z)-[1-(3,4-dimethylphenyl)-1,5-dihydro-3-methyl-5-oxo-4H-pyrazol-4-ylidene]hydrazino]-2'-hydroxy-[1,1'-biphenyl]-3-carboxylic acid can be prepared by direct compression using at least one excipient. The solid oral composition has low breakage, high strength and is stable.

According to one embodiment of the present invention is solid oral composition comprising 5-40% by weight of 3'-[(2Z)-[1-(3,4-dimethylphenyl)-1,5-dihydro-3-methyl-5-oxo-4H-pyrazol-4-ylidene]hydrazino]-2'-hydroxy-[1,1'-biphenyl]-3-carboxylic acid and at least one excipient; prepared by direct compression.

The solid oral composition may be selected from pills, pellets, powders, tablets, capsules and the like.

The pharmaceutical excipient used in the composition of the present invention may be selected from diluents, disintegrants, binders, lubricants, surfactants, glidants, sweeteners, anti-tacking agents, coloring agents and flavoring agents.

The diluents may be selected from microcrystalline cellulose, lactose (anhydrous and monohydrate), combinations of crystalline cellulose with lactose (brand names - Cellactose, Tabletosse), guar gum, silicified cellulose, corn starch, maize starch, starch derivatives such as pregelatinized starch, calcium hydrogen phosphate in anhydrous and hydrated form, sugar, fructose, dextrates, sugar alcohols such as mannitol, sorbitol, maltitol, xylitol, lactiol, and/ or other sugars such as saccharose, raffinose, trehalose, fructose or mixtures thereof, calcium carbonate, calcium lactate and/or mixtures thereof. The diluent may be in an amount of 10 to 99 weight%, preferably 25 to 90 weight% of the weight of the composition.

The pharmaceutical composition according to the invention preferably comprises one or more diluents selected from the group consisting of microcrystalline cellulose, lactose monohydrate, mannitol and mixtures thereof. Furthermore, it is preferred that the pharmaceutical composition according to the invention comprises 10 to 99 wt.-%, more preferably 25 to 90 wt.-%, most preferably 40 to 70 wt % of diluent, based on the total weight of the pharmaceutical composition.

The disintegrants may be selected from crospovidone, starch, starch derivatives such as pregelatinised starch and sodium starch glycollate, microcrystalline cellulose, carboxymethylcellulose sodium (CMC-Na, croscarmellose sodium) or calcium (CMC-Ca), cross-linked CMC-Na, polacrilin potassium, low substituted hydroxypropyl cellulose and/or mixtures thereof and can be present in an amount of 1 to 50 weight%. preferably 2 to 45 weight% based on the weight composition. Preferably, the composition of the present invention comprises croscarmellose sodium as disintergant.

The binders may be selected from polyvinylpyrrolidone, microcrystalline cellulose, cellulose ether, hydroxyethyl cellulose, hydroxypropy1 cellulose, hydroxypropy1 methylcellulose, corn starch, maize starch, pregelatinised starch, polymethacry1ate, or mixtures thereof. The binder can be present in a range of 0.5 to 25 weight%, preferably 1 to 20 weight% on the weight of the composition.

The lubricants may be selected from stearic acid, magnesium stearate, magnesium palmitate, magnesium oleate, magnesium lauryl sulfate, hydrogenated vegetable oil, hydrogenated castor oil, talc, sodium stearyl fumarate, glyceryl behanate, macrogols and/or mixtures thereof and can be present in a range of 0.1 to 10 weight%, preferably 0.5 to 5 weight% based on the weight of the composition. Preferably, the pharmaceutical composition comprises a lubricant selected from the group consisting of magnesium stearate and sodium stearyl fumarate.

The surfactants may be selected from anionic surfactants such as sodium lauryl sulfate and docusate sodium, cationic surfactants such as cetrimide, ampholytic surfactants such as N-dodecyl-N,N-dimethylbetaine, non-ionic surfactants such as sorbitan fatty acid esters (e.g. Spans^{®}), polysorbates (e.g. polyoxyethylene alkyl ethers, poloxamers, medium-chain triglycerides, polyoxylglycerides, polyoxyethylene castor oil derivates (e. g. Cremophor^{®}) and/or mixtures thereof & can be present in a range of 0.1 to 10 weight%, preferably 0.5 to 5 weight% based on the weight of the composition.

The glidants may be selected from colloidal silicon dioxide, talc, stearic acid, palmitic acid, polyethylene glycol, carnauba wax and/ or mixtures thereof and can be present in a range of 0.1 to 10 weight%, preferably 0.5 to 5 weight% based on the weight of the composition.

The sweetener can be selected from acesulfame K, sucralose, alitame, aspartame, saccharin sodium, dipotassium glycyrrhizinate, thaumatin and the like.

The coloring agents may be selected from natural pigments and inorganic materials.

The flavoring agents may be selected from natural or synthetic materials, such as orange, spearmint, strawberry and cream flavour and the like.

The solid oral composition of the present invention may be film coated.

The film coating materials may be selected from hydroxypropyl cellulose, hydroxypropyl methylcellulose, ethylcellulose, povidone and copovidone, graft copolymers of polyethyleneglycol and polyvinyl alcohol (Kollicoat IR), shellac, polymers of methacrylic acid or methacrylic acid esters, methacrylic acid-methyl acrylate copolymers, polyvinyl alcohol, plasticizers such as propylene glycol, polyethylene glycol, glycerol triacetate, triethyi citrate, antitacking agents such as talc, glycerol monostearate, magnesium stearate and colorants or pigments such as titanium dioxide or iron oxide.

The solid oral composition of the present invention has friability ranging from 0.1 to 1% preferably less than 0.5% when tested by method as described in European Pharmacopoeia chapter 2.9.7 (friability of uncoated tablets).

The solid oral composition of the present invention has hardness ranging from 40 to 150N, preferably between 70 to 150N when tested by method as described in European Pharmacopoeia chapter 2.9.8 (resistance to crushing of tablets).

The solid oral composition of the present invention has disintegration time ranging from 0.2 to 10 minutes when tested by method as described in European Pharmacopoeia chapter 2.9.1.

The solid oral composition of the present invention displays immediate release of eltrombopag.

The term "immediate release" as described herein refers to release of the active ingredient immediately on reaching the stomach. The immediate release composition of the present invention may be film coated which provides improved surface smoothness and color, increased chemical and physical stability of eltrombopag olamine due to reduced permeability of oxygen and/ or water vapor, less disintegration of the solid composition in acidic medium resulting in decreased gastrointestinal side effects, and easier swallowing of tablet.

The solid oral compositions of 3'-[(2Z)-[1-(3,4-dimethylphenyl)-1,5-dihydro-3-methyl-5-oxo-4H-pyrazol-4-ylidene]hydrazino]-2'-hydroxy-[1,1'-biphenyl]-3-carboxylic acid bis-(monoethanolamine) of the present invention have a dissolution profile wherein at least 90% release of 3'-[(2Z)-[1-(3,4-dimethylphenyl)-1,5-dihydro-3-methyl-5-oxo-4H-pyrazol-4-ylidene]hydrazino]-2'-hydroxy-[1,1'-biphenyl]-3-carboxylic acid within 45 minutes using dissolution method with 0.5% Tween 80, pH 6.8, paddle speed of 50 rpm.

The solid oral compositions of 3'-[(2Z)-[1-(3,4-dimethylphenyl)-1,5-dihydro-3-methyl-5-oxo-4H-pyrazol-4-ylidene]hydrazino]-2'-hydroxy-[1,1'-biphenyl]-3-carboxylic acid bis-(monoethanolamine) of the present invention are stable when stored at 25°/60% RH for 3 months with total impurity less than 0.5% by weight when measured by HPLC.

The solid oral compositions of 3'-[(2Z)-[1-(3,4-dimethylphenyl)-1,5-dihydro-3-methyl-5-oxo-4H-pyrazol-4-ylidene]hydrazino]-2'-hydroxy-[1,1'-biphenyl]-3-carboxylic acid bis-(monoethanolamine) of the present invention are also stable when stored at 40°/75% RH for 1 month with total impurity less than 0.5% by weight when measured by HPLC.

Typically the process for the preparation of the solid oral composition of the present invention may comprise
(a) dispensing 3'-[(2Z)-[1-(3,4-dimethylphenyl)-1,5-dihydro-3-methyl-5-oxo-4H-pyrazol-4-ylidene]hydrazino]-2'-hydroxy-[1,1'-biphenyl]-3-carboxylic acid bis-(monoethanolamine) and at least one excipient;
(b) sieving 3'-[(2Z)-[1-(3,4-dimethylphenyl)-1,5-dihydro-3-methyl-5-oxo-4H-pyrazol-4-ylidene]hydrazino]-2'-hydroxy-[1,1'-biphenyl]-3-carboxylic acid bis-(monoethanolamine) and diluents;
(c) adding optionally sieved disintegrants, binders, lubricants, surfactants, glidants, sweeteners, anti-tacking agents, coloring agents and flavoring agents and mixing;
(d) optionally lubricating;
(e) compressing and optionally film coating.

The following examples illustrate preferred embodiments in accordance with the present invention without limiting the scope of the invention.

### EXAMPLES

### Examples 1-4

| **Example No** | **1** | | **2** | | **3** | | **4** | |
|---|---|---|---|---|---|---|---|---|
| **Composition** | **mg/tab** | **%** | **mg/tab** | **%** | **mg/ta b** | **%** | **mg/ta b** | **%** |
| **Eltrombopag Olamine** | 95.70 | 9.11 | 95.70 | 19.94 | 95.70 | 19.94 | 95.70 | 22.79 |
| Mannitol 160C | 366.00 | 34.86 | - | | - | - | - | |
| Mannitol SD 200 | - | - | 310.00 | 64.58 | - | - | - | |
| Povidone K30 | 105.30 | 10.03 | - | | - | - | - | |
| Kollidon VA64 | - | - | 46.80 | 9.75 | - | - | - | |
| Lactose SD | | | | | | | 98.60 | 23.48 |
| Lactose monohydrate | | | | | 140.00 | 29.17 | - | |
| Starch 1500 | | | | | | | 42.00 | 10.00 |
| MCC 102 | 420.00 | 40.00 | - | - | 216.00 | 45.00 | 146.20 | 34.81 |
| Sodium starch glycolate | 21.00 | 2.00 | 9.50 | 1.98 | 10.30 | 2.15 | 34.00 | 8.10 |
| *Pre-lubrication Weight* | *1008.00* | *96.00* | *462.00* | 96.25 | *462.00* | 96.25 | *416.50* | 99.17 |
| Sodium stearyl fumarate | 42.00 | 4.00 | 18.00 | 3.75 | 18.00 | 3.75 | - | - |
| Magnesium stearate | | | | | | | 3.50 | 0.83 |
| ***Core Tablet Weight*** | **1050.00** | **100.00** | **480.00** | **100.00** | **480.00** | **100.00** | **420.00** | **100.00** |
| Hardness (N) | 128 | | 47 | | 41 | | 98 | |
| DT (min:s) | 07:44 | | 09:18 | | 00:25 | | 01:42 | |
| Friability (%) | 0.21 | | 0.22 | | 0.24 | | 0.18 | |

### Process:

1. The above ingredients are accurately dispensed.
2. The appropriate quantities of the excipients (Mannitol SD 200, Mannitol 160C, Povidone K30, Kollidon VA64, Lactose SD, Lactose monohydrate Starch, MCC and sodium starch glycolate) used for the preparation of the pre-lubricated blend of each example 1 - 4, are sieved through # 30 mesh and mixed with the entire quantity of eltrombopag olamine (also sieved through # 30 mesh) - Blend A
3. The appropriate quantity of Sodium stearyl fumarate is sifted through #40 mesh and is mixed with Blend A to obtain lubricated blend.
4. The lubricated blend was compressed into tablets

### Dissolution profiles and assay of compositions prepared in Examples 1-4

| **Batch No** | **1** | **2** | **3** | **4** |
|---|---|---|---|---|
| **Time** | **Drug release (%)** | | | |
| 10 min | 55.4 | 35.2 | 28.9 | 41.3 |
| 15 min | 60.7 | 46.3 | 38.1 | 50.1 |
| 30 min | 69.9 | 76.3 | 55.3 | 65.4 |
| 45 min | 74.5 | 90.0 | 64.6 | 73.1 |
| 60 min | 77.7 | 93.6 | 70.7 | 77.6 |
| **Assay** | **95.3%** | **98.2%** | **93.5%** | **95.5%** |

### Examples 5 - 6:

| **Batch No** | **5** | | **6** | |
|---|---|---|---|---|
| **Composition** | **mg/tab** | **%** | **mg/tab** | **%** |
| **Eltrombopag Olamine** | 95.70 | 22.79 | 63.80 | 22.79 |
| Mannitol SD 200 | 221.40 | 52.71 | 147.60 | 52.71 |
| Starch 1500 LM | 42.00 | 10.00 | 28.00 | 10.00 |
| Colloidal silicon dioxide | 6.30 | 1.50 | 4.20 | 1.50 |
| Sodium starch glycolate | 42.00 | 10.00 | 28.00 | 10.00 |
| *Pre-lubrication Weight* | *407.40* | *97.00* | *271.60* | *97.00* |
| Sodium stearyl fumarate | 12.60 | 3.00 | 8.40 | 3.00 |
| ***Core Tablet Weight*** | **420.00** | **100.00** | **280.00** | **100.00** |
| Hardness (N) | 138.0 | | 145.0 | |
| DT (min:s) | 02:03 | | 02:25 | |
| Friability (%) | 0.21 | | 0.18 | |

### Process:

1. The above ingredients are accurately dispensed.
2. The appropriate quantities of Mannitol SD 200 and Starch are sieved through # 30 mesh and mixed with the entire quantity of eltrombopag olamine (also sieved through # 30 mesh) - Blend A
3. The appropriate quantities of Mannitol SD 200, colloidal silicon dioxide and sodium starch glycolate are passed through # 30 mesh and the obtained blend is added to blend A and mixed - Final blend
4. The appropriate quantity of Sodium stearyl fumarate is sifted through #40 mesh and is mixed with Final blend to obtain lubricated blend which is compressed into tablets.

### Dissolution profiles and assay of compositions prepared in Examples 5 - 6

| **Batch No** | **5** | **6** |
|---|---|---|
| **Time** | **Drug release (%)** | |
| 10 min | 47.8 | 52.4 |
| 15 min | 63.5 | 65.7 |
| 30 min | 84.8 | 86.4 |
| 45 min | 92.0 | 93.9 |
| 60 min | 95.1 | 96.8 |
| **Assay** | **98.8%** | **99.8%** |

### Example 7

| **Batch No** | 7 | |
|---|---|---|
| **Composition** | **mg/tab** | % |
| **Eltrombopag Olamine** | 63.80 | 22.79 |
| Mannitol SD 200 | 144.80 | 51.71 |
| Starch 1500 LM | 28.00 | 10.00 |
| Colloidal silicon dioxide | 4.20 | 1.50 |
| Sodium starch glycolate | 30.80 | 11.00 |
| *Pre-lubrication Weight* | *271.60* | *97.00* |
| Sodium stearyl fumarate | 8.40 | 3.00 |
| ***Core Tablet Weight*** | **280.00** | **100.00** |
| Hardness (N) | **130.0** | |
| DT (min:s) | **01:22** | |
| Friability (%) | **0.15** | |
| ***Coated Tablet Weiqht*** | **288.40** | |
| Hardness (N) | **146** | |
| DT (min:s) | **02:01** | |

### Process :

1. The above ingredients are accurately dispensed.
2. The appropriate quantities of Mannitol SD 200 and Starch are sieved through # 30 mesh and mixed with the entire quantity of eltrombopag olamine (also sieved through # 30 mesh) - Blend A
3. The appropriate quantities of Mannitol SD 200, colloidal silicon dioxide and sodium starch glycolate are passed through # 30 mesh and the obtained blend is added to blend A and mixed - Final blend
4. The appropriate quantity of Sodium stearyl fumarate is sifted through #40 mesh and is mixed with Final blend to obtain lubricated blend which is compressed into tablets.
5. The tablets are further coated with HPMC based Opadry to obtain film coated tablets.

### Stability Data: for example 7 composition

| **Batch** | **FC tablets** | **FC tablets** |
|---|---|---|
| | **3 Months at 25°/60% RH** | **1 Month at 40°/75% RH** |
| **Time** | Drug Release (%) | |
| 10 min | 52.1 | 55.2 |
| 15 min | 64.5 | 69.8 |
| 30 min | 83.3 | 87.2 |
| 45 min | 90.5 | 92.8 |
| 60 min | 93.5 | 95.2 |
| **Assay (%)** | **98.5** | **100.1** |

| **Impurities** | | |
|---|---|---|
| Amino impurity (%) | <LOQ | <LOD |
| Methoxy impurity (%) | 0.00 | 0.00 |
| Methyl ester impurity (%) | 0.03 | 0.03 |
| Chloro impurity (%) | 0.02 | 0.02 |
| Max Unknown (%) | 0.04 | 0.03 |
| **Total (%)** | **0.22** | **0.21** |

### Example 8: Comparative example : Composition prepared by wet granulation process

| **Batch No** | **8** | |
|---|---|---|
| **Composition** | **mg/tab** | **%** |
| **Eltrombopag Olamine** | 95.70 | 27.34 |
| MCC 101 | 44.70 | 12.77 |
| Mannitol 160C | 89.20 | 25.49 |
| *Binding liquid* | | |
| Povidone K30 | 9.60 | 2.74 |
| *Purified water* | *q.s.* | |
| *Intragranular weight* | *239.20* | *68.34* |
| MCC 102 | 79.30 | 22.66 |
| Sodium starch glycolate | 28.00 | 8.00 |
| *Pre-lubrication Weight* | *346.50* | *99.00* |
| Maqnesium stearate | 3.50 | 1.00 |
| ***Core Tablet Weight*** | **350.00** | **100.00** |
| Hardness (N) | **55** | |
| DT (mins) | **09:44** | |
| Friability (%) | **0.31** | |

### Process :

1. The above mentioned ingredients are accurately dispensed.
2. The appropriate quantities of MCC 101 and Mannitol 160C are sieved through # 30 mesh and mixed with the entire quantity of eltrombopag olamine (also sieved through # 30 mesh) - Blend A
3. All the quantity of Povidone K30 is dissolved in purified water with mild stirring to obtain a homogenous solution - Solution A
4. Wet granulate blend A with solution A in a lab scale high shear granulator.
5. Dry the granules in tray oven at 60°C and size the dried granules in # 30 mesh.
6. The extragranular excipients MCC 102 and sodium starch glycolate are passed through # 30 mesh and the blend is mixed with the granules of step 5 to obtain the final blend.
7. The appropriate quantity of Magnesium stearate is sifted through #40 mesh and mixed with final blend and compressed into tablets.

### Comparison of Dissolution profiles and assay of compositions prepared in Examples 6, 7 and 8

| **Example** | **6** | **7 uncoated tablets** | **7 film coated tablets** | **8 uncoated tablets** | **Revolade^{™} 50 mg** |
|---|---|---|---|---|---|
| **Time** | **Drug Release (%)** | | | | |
| 10 min | 52.4 | 60.3 | 50.7 | 45.3 | 44.43 |
| 15 min | 65.7 | 71.7 | 65.8 | 65.5 | 62.03 |
| 30 min | 86.4 | 86.2 | 85.9 | 95.6 | 83.04 |
| 45 min | 93.9 | 95.0 | 91.8 | 100.2 | 87.99 |
| 60 min | 96.8 | 97.9 | 95.3 | 102.3 | 89.60 |
| **Assay** | **99.8%** | **102.4%** | **102.4%** | - | **98.4%** |

### Typical physicochemical properties of Revolade^{™} 50 mg (batch no BLH14) when measured by test methods described herein

Disintegration Time: 7 minutes 3 seconds
Hardness: 182 N
Friability: N/A (the product is film-coated and the friability has not been measured)

## Claims

1. A solid oral composition comprising 5-40% by weight of 3'-[(2Z)-[1-(3,4-dimethylphenyl)-1,5-dihydro-3-methyl-5-oxo-4H-pyrazol-4-ylidene]hydrazino]-2'-hydroxy-[1,1'-biphenyl]-3-carboxylic acid bis-(monoethanolamine) and at least one excipient; wherein the composition is prepared by direct compression.

2. A solid oral composition as claimed in claim 1 wherein the solid oral composition is selected from pills, pellets, powders, tablets, capsules and the like.

3. A solid oral composition as claimed in claim 1 wherein the excipient is selected from diluents, disintegrants, binders, lubricants, surfactants, glidants, sweeteners, anti-tacking agents, coloring agents and flavoring agents.

4. A solid oral composition as claimed in claim 1 wherein the solid oral composition is optionally film coated.

5. A solid oral composition as claimed in claim 1 wherein the solid oral composition has friability below 0.5% when tested by method as described in European Pharmacopoeia chapter 2.9.7.

6. A solid oral composition as claimed in claim 1 wherein the solid oral composition has hardness value between 70 to 150N when tested by method as described in European Pharmacopoeia chapter 2.9.8.

7. A solid oral composition as claimed in claim 1 wherein the solid oral composition has disintegration time of 0.2 to 10 minutes when tested by method as described in European Pharmacopoeia chapter 2.9.1.

8. A solid oral composition as claimed in claim 1 wherein 3'-[(2Z)-[1-(3,4-dimethylphenyl)-1,5-dihydro-3-methyl-5-oxo-4H-pyrazol-4-ylidene]hydrazino]-2'-hydroxy-[1,1'-biphenyl]-3-carboxylic acid is released immediately.

9. A solid oral composition as claimed in claim 1 wherein the composition releases at least 90% of (3'-[(2Z)-[1-(3,4-dimethylphenyl)-1,5-dihydro-3-methyl-5-oxo-4H-pyrazol-4-ylidene]hydrazino]-2'-hydroxy-[1,1'-biphenyl]-3-carboxylic acid bis-(monoethanolamine)) within 45 minutes using dissolution method with 0.5% Tween 80, pH 6.8, paddle speed 50 rpm.

10. A solid oral composition as claimed in claim 1 wherein the composition is stable for 3 months at 25°/60% RH with total impurity less than 0.5% by weight when measured by HPLC.

11. A solid oral composition as claimed in claim 1 wherein the composition is stable for 1 month at 40°/75% RH with total impurity less than 0.5% by weight when measured by HPLC.

12. A process for the preparation of a solid oral composition as claimed in the preceeding claims comprising
(a) dispensing 3'-[(2Z)-[1-(3,4-dimethylphenyl)-1,5-dihydro-3-methyl-5-oxo-4H-pyrazol-4-ylidene]hydrazino]-2'-hydroxy-[1,1'-biphenyl]-3-carboxylic acid bis-(monoethanolamine) and at least one excipient;
(b) sieving 3'-[(2Z)-[1-(3,4-dimethylphenyl)-1,5-dihydro-3-methyl-5-oxo-4H-pyrazol-4-ylidene]hydrazino]-2'-hydroxy-[1,1'-biphenyl]-3-carboxylic acid bis-(monoethanolamine) and diluents;
(c) adding optionally sieved disintegrants, binders, lubricants, surfactants, glidants, sweeteners, anti-tacking agents, coloring agents, flavoring agents and mixing;
(d) optionally lubricating;
(e) compressing and optionally film coating.
